# EUROPEAN PATENT APPLICATION

(11) **EP 3 375 587 A1**
(43) Date of publication of application: **19.09.2018**
(21) Application number: 18161957.8
(22) Date of filing: 15.03.2018
(51) Int. Cl.: B29C 43/02, B29C 47/00, B29C 47/54

(54) **PROCESSING DEVICE OF A MAKEUP PRODUCT AND MAKEUP PRODUCT MANUFACTURING METHOD**

(30) Priority: 16.03.2017 IT 201700029203
(71) Applicant: Chromavis S.p.A., 20122 Milano (IT)
(72) Inventor: Menna, Cristina, 20100 Milano (IT); Motti, Erika, 26010 Credera-Rubbiano (CR) (IT); Gaboardi, Mauro, 26026 Pizzighettone (CR) (IT)
(74) Representative: Vanosi, Adelio Valeriano

(57) **Abstract**

Processing device of a makeup product comprising:
- a first plate provided with first mutually adjacent channels with parallel axes, of semicircle section, and
- a second plate provided with second mutually adjacent channels with parallel axes, also of semicircle section, the first and the second channels being configured such that, when the first plate and the second plate are overlapped with their respective channels facing each other, each pair of first and second channels forms a tubular element with circular section;
- the device having guides configured to allow the movement of a plate relative to the other, in a direction parallel with respect to the axes of the channels; and
- at least one movement system of a plate relative to the other.

## Description

### FIELD OF THE INVENTION

The present invention relates to a device for the preparation of cosmetic products for makeup, such as eye shadows, face powders, blushers, lip colours, eyebrow and hair colours, in the form of single or multi colour spheroids.

In particular, the invention concerns a device employed in a preparation step of a product for makeup in the form of a spheroid with a solid consistency and an excellent smooth powdery coverage.

The invention also relates to the spheroids created with the said device.

### BACKGROUND ART

Currently, cosmetic products for the shape makeup are achieved using a coating pan.

A coating pan is a container with a tilted rotation axis with a flattened spheroidal shaped end and the other end with an opening to introduce the ingredients. It features a motor that allows the container to rotate on an axis that passes through the flattened end; the container is made of stainless steel (in the past it was also made of copper).

The cosmetic powder and some spherical shaped cores are placed in the container, the said cores, which may for example be made of polyethylene, trigger the formation process of the spheres through the coating of the cores with the cosmetic powder. The cosmetic powder and the cores continuously roll while solutions of water and a binding substance (for example sorbitol) are sprayed, so the spheres increase in size, up to the required diameter.

Suitable systems are envisaged to automatically spray or pour the solutions or suspensions of binding agent and a forced ventilation system (supplied with hot air) is also foreseen, which removes the humidity and favours the evaporation of the solvent, accelerating the drying process while the spheroids are being formed.

Alternatively, the product can be dried in an oven in a subsequent step.

This technology is used to obtain spheres of single colour cosmetic powder.

EP2106782-A1 describes a method of production of spheroids wherein the coating steps are achieved with different colours on concentric layers or shells. The different colours however, are not visible on the outer surface, which is always single colour.

The beads obtained with the method illustrated above do not offer a good coverage due to the high content of the binding agent required for the increase in size of the spheres. Furthermore, after a certain period of use the spherical particles used as cores are uncovered, and they can't be used as makeup even though they are made of a material that complies with the cosmetic regulations.

EP1458331-A1 describes a method to produce extruded cosmetics.

### SUMMARY

The object of the present invention is to provide an improved device to achieve makeup elements mainly in the shape of spheroids.

Another object is to provide a device that speeds up the forming operations of the spheroids made of one or more cosmetic pastes, minimising waste and obtaining a very regular spheroidal shape.

This and other objects are achieved by means of a method and equipment according to the technical teachings of the claims annexed hereto.

Advantageously, the device can be used in a production step of multi colour spheroidal makeup elements.

### BRIEF DESCRIPTION OF THE FIGURES

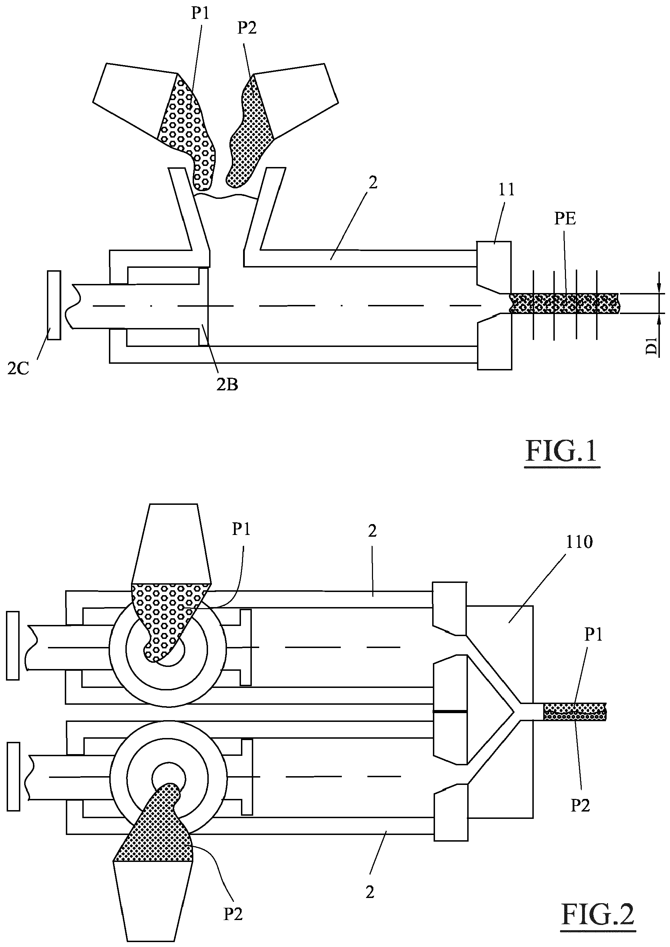

Further characteristics and advantages of the invention will become clearer in the description of a preferred but not exclusive embodiment of the method, illustrated - by way of a nonlimiting example - in the drawings annexed hereto, in which:
figure 1 shows a step of the preparation process of the spheroids according to the present invention;
figure 2 shows a step of another way of preparing the spheroids according to the present invention;
figure 3/A shows an intermediate step of the preparation process of the spheroids, which follows the one in figure 2;

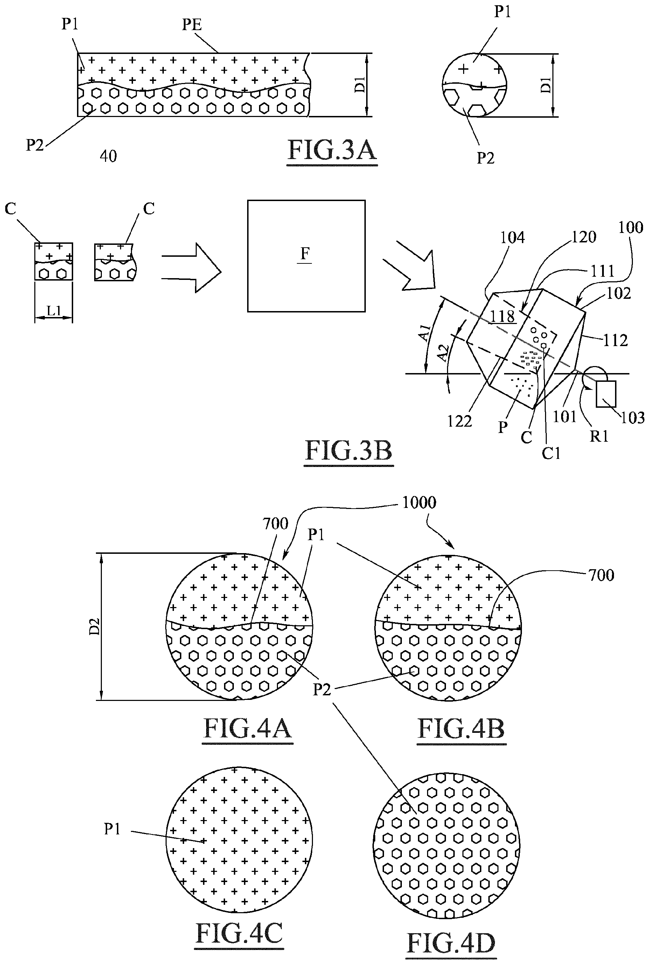

figure 3/B shows a step that follows the one in figure 3/A;
figures from 4A to 4D show respectively a front, side, top and bottom view of a makeup spheroid, achieved according to the steps in figures 2 and 3;

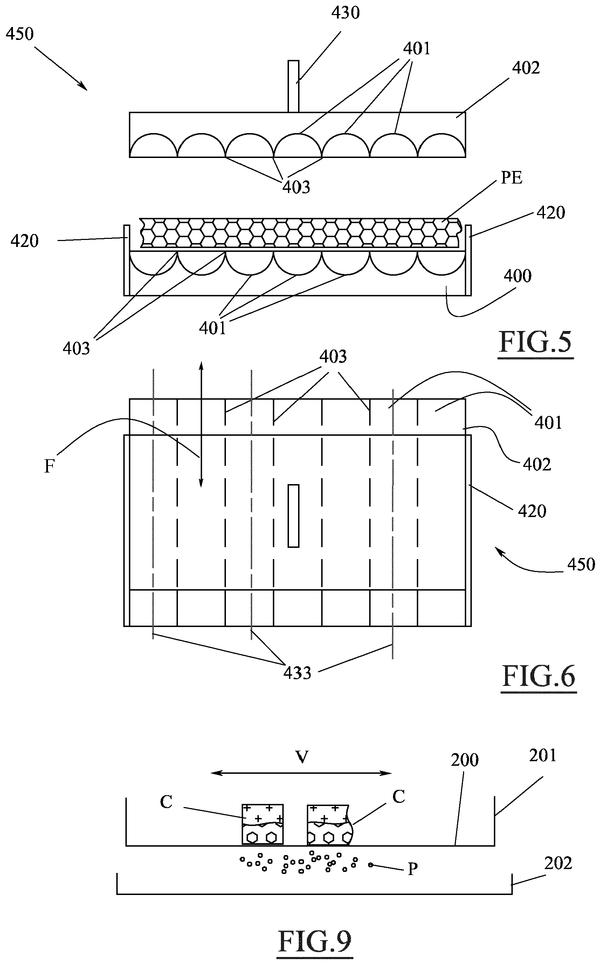

figures 5 and 6 show an alternative step to the one in figure 3, to obtain the spheroids according to the present invention: in particular, figure 5 is a front view of a device to preform the alternative step, while figure 6 is a plant view of the device of figure 5;

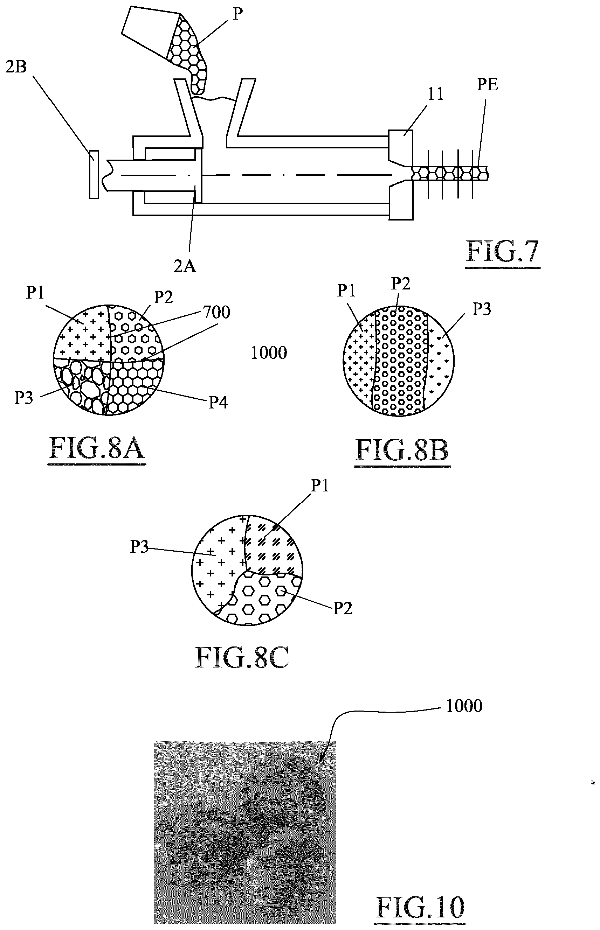

figure 7 shows another production step of the spheroids, alternative to the one in figures 1 and 2;
figures 8A and 8C show some spheroids that can be obtained with the process according to the present invention, from a front view;
figure 9 shows another alternative step to the one in figure 3, to obtain the spheroids according to the present invention;
figure 10 shows the spheroids obtained by means of the step shown in figure 1;
figures from 11 to 14 show a particular extruder, alternative to those shown in figures 1, 2 and 7.

### DETAILED DESCRIPTION

The procedure to produce the spheroids according to the present invention includes various mixing, extrusion, shaping and drying steps of a cosmetic paste. By means of this process, cosmetic products for makeup can be obtained (or makeup products) with a solid consistency and spheroidal shape.

Equipment and machines (some conventional, others innovative) for the production of the said makeup spheroids shall also be described.

In this document, the term "spheroid" is understood to mean a shape "similar" to that of a sphere or of a small ball. In fact, as it is a product for makeup mainly made of makeup powder, the final shape shall not be that of a perfect sphere and it shall feature some natural irregularities. For this reason, the "diameter" of the spheroids is understood to mean an "average" diameter thereof.

The spheroids obtained with the process have the characteristic of being easy to handle and at the same time they feature a good coverage of a "powdery" kind.

### Cosmetic paste

According to one aspect of the present invention the cosmetic paste is prepared (generally indicated as P), which is obtained by mixing some fats for cosmetic use, colouring powders and at least one solvent for cosmetic use.

More in general, the procedure foresees the preparation of a cosmetic paste P (or bulk) and can include the following steps:
a) preparation of two phases herein called "colouring powders" and "fatty emulsion";
b) mixing the aforementioned phases to obtain a cosmetic paste (bulk);

### Fatty emulsion

The "fatty emulsion", according to the present invention is obtained by processing the fats for cosmetic use with at least one solvent, for example water or any solvent suitable for cosmetic use, including mixtures thereof; a characteristic of the solvent used is that it must be possible to remove it by means of the drying process after the extrusion, at temperatures that do not alter the end product, advantageously at temperatures not greater than 50°C.

The solvent may be neutral or coloured, by virtue of its specific properties or due to the addition of colouring substances.

Water, for obvious sourcing and cost reasons, is a preferred solvent according to the present invention.

The expression "fats for cosmetic use" means any fatty matter suitable for the preparation of cosmetic products, such as for example the esters of fatty acids, triglycerides, waxes, derivatives and extracts of fruit and seed oils, etc.

Some useful fats for cosmetic use according to the invention are, for example, sorbitan stearate, isopropyl stearate, caprylic / capric triglycerides, dipentacrythrityl hexahydroxy/ stearate rosinate (sold under the brand name Cosmol 168AR), Magnesium Myristate and olive oil.

### Colouring powders

The expression "colouring powders" means any powder, or mixture of powders, that contains colouring pigments suitable for cosmetic use.

Suitable colouring powders include, for example, those obtained by mixing artificial and/or natural pigments, matte or pearly, with inert powders as diluents, such as mica or talc, in variable quantities according to the required powdery effect and colouring power.

The pearly or colouring substances that may be used include one or more of the following:
Ti02 (CI 77891) + mica (CI 77019)
Bismuth oxychloride CI 77163
Mica CI 77019
Copper and bronze powder CI 7740
Iron oxide CI 77491-2-9
Ultramarine Blue CI 77007
Manganese violet CI 77742
Hydrated chromium oxide CI 77289
Chromium oxide green CI 77288
Ferric ferrocyanide CI 77510
Titanium dioxide CI 77891
D&C red no. 7 Ca lake CI 15850:1
D&C red no. 19 Al lake CI 45170:3
D&C red no. 6 Ba lake CI 15850: 2
D&C red no. 3 Al lake CI 45430: 1
D&C red no. 9 Ba lake CI 15585: 1
D&C red no. 21 Al lake CI 45380: 3
D&C yellow no. 5 Al lake CI 19140: 1
D&C red no. 30 Al lake CI 73360
D&C yellow no. 10 Al lake CI 47005: 1
D&C red no. 27 Al lake CI 45410: 2
D&C yellow no. 5 Al lake CI 19140: 1
D&C orange no. 5 CI 45370: 1
FD&C yellow no. 6 Al lake CI 15985:1
FD&C blue no. 1 Al lake CI 42090:2
D&C red 36 CI 12085
Carmine CI 75470.

It is therefore evident, that the cosmetic paste P can be obtained by mixing each single component one after the other, for example in a mixer, or by preparing the two phases "fatty emulsion" and "colouring powders" first and then mixing them.

The quantity of the single components may vary within a wide range, depending on the type of product required.

The "fatty emulsion" and "colouring powders" phases are preferably mixed in equal quantities, that is to say about 50-50% by weight.

Other components, inert or non-inert, may be added to the colouring powders and fatty emulsion phases of the invention or directly to the paste to be extruded. For example, additives may be added, such as solid and liquid preservatives like sodium benzoate, thickeners like starch and its derivatives, diluents, gelling and binding agents in order to make the paste obtained by mixing the two phases suitable to be extruded; various inert powders may be added and any desired fragrance, etc... The said additives, as well as their properties and applications in the cosmetic fields, are well known to a person skilled in the art.

Some examples of additives are described in the experimental part.

The colouring powders are generally prepared by mixing the desired pigments with inert components such as talc, silica and mica. According to a preferred aspect, parabens and sodium dehydroacetate are added to the mixture, having the function of preserving the entire product during all the processing steps.

The said components are advantageously milled for a few minutes to obtain a very homogenous powder.

The emulsion and the colouring powders thus prepared are then processed in a mixer and transferred to an extruder; the paste comes out of the dies in the required shapes. Upon exiting the extruder, the product preferably rests on plates made of inert material, wherein inert means that it does not interact with the product, such as PVC, steel or similar.

A general procedure to prepare the cosmetic paste is described below.

All the machinery necessary for the processing procedure is cleaned and sanitised beforehand.

### Preparation of the fatty emulsion

Weigh the water in an emulsifying machine; add the chosen preservative and mix until completely dispersed. Add any thickeners and the mixture of liquid preservatives and disperse with a homogeniser. Weigh and dissolve the fatty mass (stereates and triglycerides) at about 60°C and add it to the previously prepared mixture and homogenise. Add the gelling agent and process for a few minutes. Check viscosity and pour into clean drums.

### Preparation of the colouring powders

Weigh the inert components, binders and any preservatives (talc, parabens, etc.) and mill for a few minutes. Weigh and add the raw materials that make up the desired colour and mill until a uniform mixture is obtained.

### Example of the preparation of the cosmetic paste

Mix the fatty emulsion and the colouring powders in a mixer for about 10 minutes. Transfer into clean bags and extrude using a drawing machine. Bake the extruded product for 3 days at about 50°C.

An example of a cosmetic paste is described below.

**Qualitative-quantitative composition of a "typical paste" before extrusion:**

| Water | 39.40 % |
|---|---|
| Fats for cosmetic use (mixture of isopropyl | |
| stearate, di-C12-13 alkyl malate, Cosmol 168AR) | 6.00 % |
| Pigments in mixture with mica | 38.4 % |
| Xanthan gum | 0.05 % |
| Preservatives (mixture of sorbic acid and parabens) | 1.1% |
| Inert components (mix of zea mays, nylon powder,talc) | 14.55% |
| Fragrance | 0.5 % |

### Extrusion

The extrusion process of the cosmetic paste P (or of different cosmetic pastes P1 ....Pn prepared for example as described above), can be carried out by passing the paste P through a conventional extruder 2 (of the piston type, like the one in fig. 7 or of the screw type) to obtain a semi-solid product 10, still damp, of the desired shape imposed by an extrusion head 11.

In the case shown in fig. 7 (extrusion of a single cosmetic paste), the extrusion head 11 allows to obtain an extruded paste PE with the appearance of a strip with a circular section. The extruded paste PE is therefore in the shape of a cylinder.

As already mentioned, the extruders useful in the process of the invention are, for example, of the piston (preferred) or screw type. For an industrial type production, the piston 2A (or the screw) can be driven by a suitable motor 2B, possibly controlled by an inverter.

In the case of productions of small lots, a manual extruder can be used. Substantially it is a syringe that is filled with cosmetic paste P used to feed a mechanical extruder.

The extruded paste PE exits the extruder (or the syringe) with different cross-sections according to the extrusion heads 11 used (for example, a cylinder, a parallelepiped, etc.).

In the case in which the extruder is fed with a single paste P, at the output, the extruded paste PE will be substantially single-coloured.

There are however possible variations to the above mentioned process, such as the one shown in figure 5.

In this case, the extruder is fed with at least a first paste P1 and a second cosmetic paste P2 (or bulk). The cosmetic pastes P1 and P2 differ in at least one characteristic, but are prepared according to the methods described above. Preferentially, the characteristic that differentiates paste P1 and paste P2 is only the colour, but the differences may also include other aspects of the paste composition.

The extruded paste PE as in figure 1, has a 'melange' appearance and has the purpose of producing spheroids such as those in figure 10.

Essentially, inside the extruder, the first paste P1 and the second paste P2 mix randomly with each other, but each one keeps its own identity. Essentially, by means of this procedure, an extruded paste PE is formed from a matrix (for example of the cosmetic paste P1) within which the islands formed by the cosmetic paste P2 are clearly identifiable and visible.

Obviously, in this case, the extruder will be fed with more paste P1 and with a smaller quantity of paste P2. In the case in which the extruder is fed with substantially the same quantity of pastes P1 and P2, the effect will be similar but it will no longer be possible to identify a 'matrix'.

To obtain the effect described, it is necessary to use a screw type extruder instead of a piston type one. In this way, the two pastes will mix with each other inside the extruder, improving the final appearance of the paste.

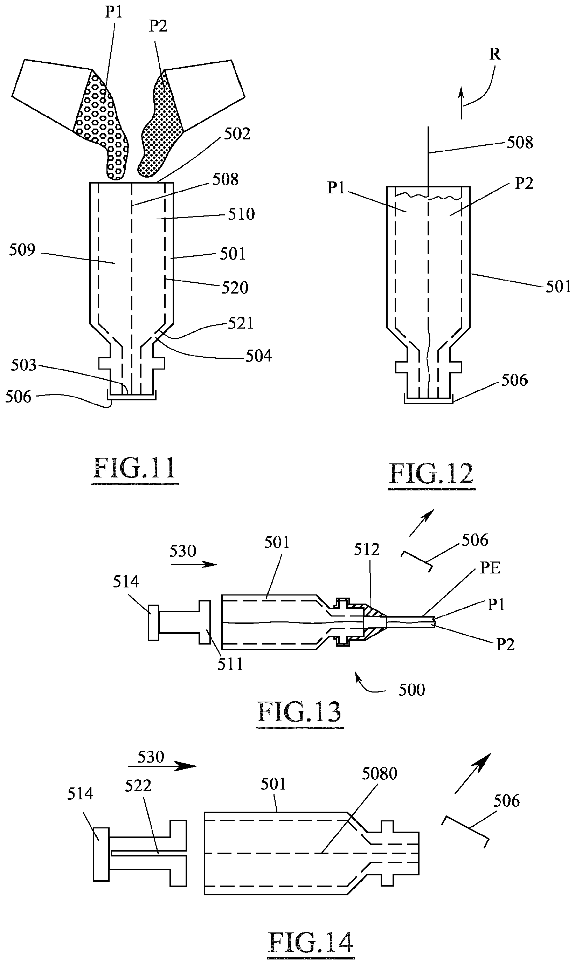

According to another variant, it is possible to roughly pre-mix (for example, by means of a planetary mixer) the two cosmetic pastes P1 and P2 and feed the mix of the two pre-mixed pastes to the extruder (piston or screw type, or to the equipment in Figure 13, which will be described below).

Another method to obtain an extruded paste PE to obtain spheroids with a different and very innovative appearance, is the one shown in Figure 2.

In this case, a pair of extruders 2 is used (similar to those already described), each one fed with a different cosmetic paste P1, P2, whose output flows into a common extrusion head 110. It is, in fact, a co-extrusion.

The common extrusion head 110 supports and "merges" the first P1 and the second paste P2 along the interface surface 700 that is planar (possibly with some irregularities), allowing to obtain an extruded paste PE, for example two-coloured, wherein the separation surface 40 (or interface surface 700, in the finished product) between the first and second paste is substantially diametrical and well marked, as can be clearly seen in Figure 3A, where the strip of extruded paste PE is shown both from a side and front view.

Obviously, in the case of a square or rectangular section (or other shape) extrusion head, the front view of the extruded paste PE will be different and corresponding to the exit section of the extruder, but the demarcation line 40 between the first P1 and the second paste P2 will be clearly visible.

An economically advantageous alternative to the extruders in Figures 1, 2 and 7 is shown in Figures 11-14; it is an innovative extrusion equipment. The apparatus 500 to produce a single or multi coloured makeup product comprises:
- a tank 501 provided at a first end with an inlet 502 and at a second end with an opening 503 at the level of a convergent part 504 of the tank, and with a removable closure 506 of the opening at the second end;
- a partition 508 configured to divide the inside of the tank into at least one first 509 and one second compartment 510, mutually insulated.

In this document, mutually insulated compartments is understood to mean that the insulation must not allow the paste to be extruded, contained in one of the compartments, to mix totally with the paste contained in the other compartment when the partition is in position.

Obviously, minimum leaks of paste are allowed, which do not however affect the quality of the final extruded product.

The apparatus 500 also comprises:
- a piston 511 that slides at least partially inside the tank 501 towards the opening 503 at the second end;
- possibly an extrusion head 512 with a shaped orifice 513, that can be combined with the opening of the second end, that is in communication with the fluid passage thereof; the extrusion head can define a section of the extrusion also different from that of the opening 503.

The apparatus 500 is fitted with:
- a movement system 514 to move the piston in the tank (for example a motor with inverter, or a manual, pneumatic or hydraulic system) to extrude a paste present in the tank at least through the opening 503 of the second end or preferably through the extrusion head 513 associated with opening 503.

It is evident that with a configuration such as the one described above, it is possible to obtain an extrusion formed by at least two cosmetic pastes mutually coupled along an interface surface 700, defined by the presence of the partition 508. Obviously if more partitions are envisaged (and therefore more compartments), the cosmetic pastes that can be co-extruded can be as many as the compartments (for example to produce the striped spheroid in figure 8).

It should be noted that the partition in the tank can extend up to the opening 503, so as to keep the pastes well separated, and preferably it must touch or be very close to the closure 506 (when it closes the opening).

The inside of the tank 501 can be defined by a first cylindrical portion 520, followed, in the direction of extrusion, by a second frustum convergent portion 521.

The partition 508 can be fixed firmly in the tank (figure 14), and in this case the piston is shaped (for example with a slit 522 to house the partition) so as to be able to slide in the tank even in the presence of the partition, or, as in Figure 11, 12 and 13 the partition 508 can be removable (Figure 12) and is removed (arrow R) before operating the piston 511 (which in this case has a section 523 similar to the internal section of the tank). During the removal, the pastes P1 and P2 (and the others that may be present in the tank) do not mix substantially, as their consistency, as said, is quite pasty; in any case, it has also been verified that by removing the partition 508 before extrusion, the interface 700 is sufficiently regular.

If necessary, the tank can be combined with an orientation system configured to position it in at least one first position (Fig. 11) in which the mouth 502 is facing up, so that the tank can be filled by gravity, and a second position wherein an axis of the tank is inclined with respect to the vertical line, preferably in horizontal position (Figures 13 and 14) so as to facilitate the extrusion and removal or arrangement of the extruded pastes.

It should be noted that in the embodiment shown, the tank is provided with a partition 508, but it is also possible to use the apparatus to carry out a simple extrusion of the two cosmetic pastes P1 and P2 mixed as described above, to obtain a melange effect (Fig. 10, or extruded paste in Figure 1).

Essentially, the apparatus 500 makes available a method to produce a multi colour makeup product, comprising the following steps:
- prepare a tank 501 provided, at one first end, with an inlet 502 for at least two cosmetic pastes and, at one second end, with an opening 503 preferably at the level of a convergent part of the tank, and temporarily close (with closure 506) the opening at the second end;
- the tank being divided into at least one first 509 and one second compartment 510, mutually insulated;
- position the tank with the first end upwards (fig. 11);
- after having positioned the tank, pour (fig. 11) a first makeup paste P1 into the first compartment 509, and a second makeup paste P2 into the second compartment 501, the first and the second makeup pastes being visually different, and proceed as described above;
- insert a piston 511 in the input mouth, that slides at least partially inside the tank and open the second end, possibly combining it with an extrusion head 512;
- move (arrow 530) the piston 511 relative to the cup-shaped body so as to extrude, at least through the opening of the second end or the extrusion head, the cosmetic pastes present inside the cup-shaped body;
- carry out a first shaping of the extrusion (for example by means of one or more of the techniques illustrated later in the dedicated chapter);
- dry the extrusion (for example in the manner illustrated later in the dedicated chapter);
- if necessary, perform a second shaping of the dried extrusion (for example, as will be described later in the dedicated chapter, to make it take on a spheroidal conformation). According to one aspect of the method, the tank can be divided into at least two compartments 509 and 510 by a removable partition 508; in this case the following further steps can be envisaged:
- before pouring the first and second cosmetic pastes, insert the partition 508 into the tank to form one first and one second compartment, and
- before inserting the sliding piston 511 in the mouth 502 of the tank 501, remove (arrow R) the partition from the tank.

For example, the extrusion head 512 may be provided with an output section of a given shape (for example, a circle, an ellipse, a square, a rectangle, a star, a flower, etc.) and the head 512 (interchangeable) may be permanently combined with the opening of the second end of the tank during an extrusion operation.

The method may foresee that, prior to extruding the pastes, the tank is positioned with a substantially horizontal axis thereof, to facilitate the extrusion.

It should be noted that the apparatus described, can be used in a production step of spheroidal makeup elements, and also to produce extruded multi colour stick-shaped pieces.

In this case, the processing steps of the extrusion will be reduced to a shaping (for example to cut the extrusion to the desired length) and to the drying of the stick-shaped pieces, according to the techniques described in the relative paragraph.

If necessary, the multi coloured stick-shaped pieces (for example "striped") made with the described apparatus, may be subjected to a step to remove any crust that may form during the drying process. This step to remove the crust, however, should avoid substantially changing the shape of the stick-shaped pieces, and can be carried out using all the methods conventionally used for single colour extruded stick-shaped pieces.

### First shaping

According to an aspect of the innovation, once the extrusion step is finished and the extruded paste PE has been obtained by means of one of the methods described above (or those that will eventually be described below), a first shaping of the extrusion can be carried out.

The first shaping can take place, for example, by simply cutting the extruded paste PE in many intermediate elements C, in the form of small cylinders (cubes or parallelepipeds), for example by slicing the extrusion with a blade or in another known manner.

Preferably, the length L1 of the intermediate elements C will be equal to or slightly greater than its diameter D1 (if the extrusion opening has a circular section). If instead, the head of the extruder has a square section, the length L1 of the cylinders will be equal to or slightly greater than the side of the square defined by the output section of the extruder. In this case small cubes will be produced (possibly with rounded corners).

According to a different technique, the extruded paste PE just exited from the extruder and still damp, can undergo a cutting process by means of an apparatus 450, different from the one illustrated above.

With the cutting apparatus 450 (or better "device"), the extruded paste PE (as described above) is placed on a first plate 400 provided with a plurality of side-by-side channels and with a semicircular cross-section. The damp extruded mixture is positioned (or directly extruded) orthogonally to the channels of the first plate 400.

A second plate 402 is also foreseen, very similar to the first one, also provided with channels 401 with a semicircular section. See figure 5 where the plates 400, 402 are represented in a simplified front view.

The first plate 400 and the second plate 402 are then moved close to each other (arrow F of fig.6, where the plates 400, 402 are represented in a simplified top view) so as to close onto the extruded paste PE, which is cut by the edges 403, which separate the channels 401 of each plate, at the level of a free surface thereof.

In addition to cutting, the apparatus 450 also pre-shapes in a spherical shape the cut portions of extruded paste.

As soon as the cut is made, with the two overlapping plates (and possibly kept aligned by suitable edges 420 joined onto one of the plates), a plate is moved (possibly with reciprocating motion) relative to the other (for example the second plate 402 with respect to the first), advantageously by means of a handle 430, connected to the second plate 402.

The extruded paste PE, sectioned and trapped in the channels, is then shaped in a substantially spherical shape imposed by the shape of the channels and by the sliding movement of the plates.

This is due to the friction between the paste PE and the walls of the channels, that impose a rolling movement to the sectioned paste. In other words, the paste trapped in one channel, is dragged by the movement of the plates, that makes it roll within the channel.

It has been verified that the relative movement between the paste PE and the plates it is enough to prevent the sticking of the paste to the walls of the channels. If a sticking of small parts of paste occurs to the walls of a channel, the stuck small parts are then incorporated in the sphere formed by said channel, once the sphere rolls on the stuck small part, by virtue of the movement of the plates that drags the spheres.

In order to minimize a possible sticking of the paste to the walls of the channels, a lubricant fluid (that may be silicon based) may be distributed on the channels before placing the extruded paste between the plates. Those kinds of lubricants are well known in the field of the production of cosmetics, and are for example, wide used to prevent sticking of lipsticks to silicon moulds.

As an alternative, an anti-sticking coating may be present on the walls of the channels of both plates. Suitable anti-sticking coating may comprise Teflon or other suitable materials.

In this way intermediate elements C1 can be produced, already pre-shaped as spheres.

This process significantly reduces waste (compared to the creation of a spherical shape starting from cylinders / cubes / parallelepipeds), thus improving productivity.

In addition, it makes it possible to obtain a more regular spherical shape at the end of the process.

Obviously the opening of the plates, their closure and their reciprocal movement in an axial direction with respect to the channels 401, can be easily mechanised, making a first shaping machine usable at industrial production level, for large batches.

Even the unloading of the formed beads C1 can be mechanised, for example by tilting the first plate 400 following the forming operations, or by arranging a movable rod-like element on the fixed plate which pushes the beads towards a collection zone.

Furthermore, the automatic sliding of the plate in front of the extrusion head, can allow the automatic positioning of the extruded paste PE on the first plate 400.

### Drying

After having obtained the intermediate elements C and C1, in the manner described, which may have the C shape given by the first shaping method (small cylinders, cubes or parallelepipeds etc.) or the C1 shape given by the second first shaping method (very regular beads), they can be dried.

The drying step of the process can be performed according to conventional techniques, for example: in a conventional oven F, in a vacuum oven, in a fluidised bed furnace, in the air, at room temperature etc. provided that this procedure creates conditions that ensure the evaporation of almost all the solvent, whatever it may be, used to prepare the "paste" to be extruded.

Obviously lower temperatures require longer drying times whereas at higher temperatures drying times will be shorter. The drying (or baking) will be preferably performed at low temperatures in order to permit slow elimination of the solvent from the paste and to obtain a well-pressed and uniform end product, without altering the end product.

Advantageously, the drying is performed in an oven at temperatures of 35-65°C, around 50°C for example, until almost complete evaporation of the solvent, for example until the residual humidity is below or equal to 5%.

Even if the utmost attention is paid during the drying step, the dried intermediate elements C may have a crust (more or less thick), which must be removed before use to improve the coverage of the end makeup product.

### Second shaping

According to one aspect of the present invention, the intermediate elements C and C1, produced according to what has been described up to now and dried, are then processed to remove the dried surface layer and possibly other material present, in order to give them a shape that is as much as possible spheroidal.

The second shaping can be obtained automatically through the use of the machine 100 shown in figure 3.

The machine 100 of the present invention comprises a cup-shaped body 102 hinged along an axis 101. The axis is preferably inclined and the cup-shaped body 102 is torsionally connected to a motor 103, which controls its rotation R about the axis 101.

The cup-shaped body 102 can have a spheroidal conformation with an opening 104 for the introduction of the products to be processed, similar to that of a conventional coating pan. In this case, the axis 101 may coincide with the axis of the sphere passing through the centre of the opening 104. Alternatively, the cup-shaped body 102 may have a shape as shown in figure 3B, that is with a central portion 110 substantially cylindrical, connected to a truncated cone shaped portion 111 defining the opening 104, and a substantially closed conical portion 112 which defines a bottom of the cup-shaped body 102. This shape is similar to that of a cement mixer.

In a simplified form, the cup-shaped body can simply have a cylindrical shape.

According to an aspect of the invention, at least one internal surface of the cup-shaped body 102 comprises an abrasive element.

The abrasive element may comprise an abrasive coating inside the cup-shaped body 102, or, in a preferred embodiment, a basket 120 (cylindrical or frustum-shaped) with perforated abrasive walls 122 (or mesh, for example made of metal, fixed in such a way as to rotate around its own axis of symmetry. The basket 120 may be cylindrical or frustum-shaped, and must be provided with a bottom 121 connected to the side walls 122. Advantageously, also the bottom 121 may be perforated, and advantageously it can be made of the same abrasive material as the side walls 122.

According to an advantageous embodiment, the basket 120 is fixed to a mouth 104 of the cup-shaped body 102 of a coating pan and rotates integrally with it, around the axis 101 which may coincide with the axis of the basket 120.

In this embodiment, the dried intermediate elements C and C1 are transferred inside the cup-shaped body 102, and specifically inside the basket 120.

In this way, the rotation along an inclined axis 101 of the basket 120 causes the friction of the intermediate elements C against the abrasive perforated or mesh walls thereof. The friction originates from two contrasting forces; the effect of gravity, which tends to bring the elements C back to the lower part of the basket, and the dragging effect originated by the inclined rotation of the walls of the basket 120, which tends to bring the elements C back to a higher position compared to the bottom.

Such friction on the walls of the basket 120 smoothes the dried intermediate elements C, removing any surface crust formed during drying in the oven (thus shaping them).

If the dried intermediate elements C, originally introduced into the basket 120, did not already have a substantially spherical shape, the rotation of the basket is prolonged for the time necessary to remove part of the material thereof, until a spheroidal shape is obtained for each of the intermediate elements C processed in this way.

In the illustrated configuration, the cup-shaped body of the coating pan, as can be seen, has the sole purpose of collecting the dust P generated by the friction on the walls of the basket 120, keeping it substantially separate from the intermediate elements C being processed.

Advantageously, in the described embodiment, the basket 120 rotates integrally with the coating pan.

Obviously, the only important feature to obtain the processing as described above is that the basket 120, comprising the abrasive walls, can rotate around an axis with inclination A1 with respect to the horizontal line. In particular, the axis 101 can be inclined so that the elements C contained inside the basket 120 do not protrude from the open side of the basket during rotation, or rather, so that the walls 122 are also inclined at an angle A2 with respect to a horizontal plane (for example with respect to the floor). Preferably, the inclination A1 of the rotation axis 101 with respect to the horizontal plane (for example that of the floor) can be comprised between 30 and 70°, while the inclination A2 of the walls 122 is less than or equal to Al.

In an alternative embodiment, it is the inside of the cup-shaped body 102 of the coating pan that can be coated with an abrasive material. In this way the cup-shaped body 102 of the coating pan becomes functionally equivalent to the basket 120 described above. In this case, the mere rotation of the cup-shaped body 102 allows the processing of the intermediate elements C in a manner similar to that described above.

The only difference is that, in this configuration, the dust P that is created during the processing remains and rotate 'together' with the intermediate elements C to be processed, reducing the effectiveness of the process. It is therefore useful to envisage suitable means to remove it (for example suction devices).

It should be note, that processing using the machine described above, allows to obtain spheroids with a good spherical shape, and all with quite homogeneous dimensions (average diameters).

Another method to process the intermediate elements C and C1 to obtain a substantially spherical shape, even if less 'precise' than that obtainable with the previous method, is that of arranging the dried intermediate C elements on a mesh vibrating screen (fig. 9). This method works very well for intermediate elements C1, which already have a substantially spherical shape, and therefore those made using the first shaping device 450.

Essentially, the intermediate elements C and C1 are loaded onto a mesh 200 provided with sides 201 to which a mechanical vibratory movement V is applied, which makes the elements roll, rounding them and at the same time cleaning them from the crust formed during the possible baking in the oven. The removed dust P goes through the wire mesh 200 and is collected in a bag 202.

The different steps of the process described, therefore make it possible to obtain spheroidal makeup elements 1000 with the following characteristics: optimal consistency permitting direct or indirect application, excellent coverage effect and, at the same time, a creamy "finish" even though the products are powder-based.

In addition to what has already been said, and to the already evident advantages deriving from the new process described above, the following should be noted.

The spheroidal makeup elements 1000 obtained according to some steps of the method described above, and possibly with the aid of the machine 100, of the device 450 and of the apparatus 500, are particularly innovative with respect to those currently on the market. In fact, as specified, the commercially available spheroidal makeup elements (beads) have a uniform colouring, or are formed by two concentric shells of different colours, containing a non-makeup nucleus (example: plastic).

According to some aspects of the present invention, for example with an extrusion such as that shown in figure 1, it is possible to make makeup spheroids with a 'melange' effect.

Indeed, on the surface of the makeup element there are two distinct colours distributed randomly in island-like shapes on the surface of the bead. Figure 10 shows an example. In addition, the two-colour "patchy" texture remains visible in a substantially unchanged manner even when the small balls are used for makeup, that is when a surface layer thereof is removed to be used to put on makeup.

Until now, on the market, there have not been any makeup cosmetics in the form of beads or spheroids, with such an innovative texture.

The same result can be obtained using the extruder in figures 11-13, without the partition 508 and fed with the first and the second paste, premixed as described above (or possibly also with other pastes).

By using instead the co-extrusion shown in figure 2, it is possible to obtain beads (or spheroidal makeup elements 1000), made with at least two pastes P1 and P2 (for example of different colours or with other differentiating characteristics) so that the spheroidal surface features at least two pastes arranged in a homogeneous and orderly manner. See figures 4A-4D. In the said figures, a hemisphere is made of makeup paste P1 and the other is made of makeup paste P2 (extruded and dried).

The said spheroidal element 1000, can also be obtained starting from the co-extruder of figures 11-14, as explained above.

By way of example, the order of preparation and mixing of the ingredients to make the cosmetic paste, as well as some of the steps of the described processes, can be arbitrarily modified, obtaining nevertheless products with the same characteristics.

One or more embodiments of the invention or of the method subject matter of the present invention, may comprise one or more of the following characteristics or steps, alone or combined.

The invention provides a spherical makeup element 1000 consisting of at least a first P1 and a second makeup product P2, the first and the second makeup products being visually different, the first and the second makeup product being simultaneously visible on the outer surface of the spheroid (fig 10).

According to one aspect, the first and the second makeup products appear on the outer surface of the spheroidal makeup element in each state of consumption of the makeup element deriving from its use (fig. 10 - fig. 4A-4D, fig. 8A-8C).

According to a further aspect, an interface surface 700 between the first and the second makeup product has at least one planar part, possibly irregular or in which the interface surface between the first and the second makeup product has a planar, possibly irregular surface (as can be seen from figures 8A, 8B and 8C, which are obtained respectively by means of the suitably configured extruders in figure 2, or by means of the apparatus 500 where a cross-shaped partition, a flat partition and a Y-shaped partition have been respectively used, as well as the necessary number of cosmetic pastes).

In the present text "planar" and "possibly irregular" development, mean that the interface surface, although having some irregularity due to the processing method, extends substantially along a plane, and not for example, along a sphere or other geometric figure.

Essentially, the interface surface, with the exception of possible irregularities with non-periodic distribution, is at a first approximation similar to a plane.

According to one aspect, an interface surface between the first and the second makeup product has a development similar to the lateral surface of a prism, possibly irregular, and / or wherein the cross-sections of the prism have a circular shape (the prism is in fact a cylinder) or polygonal or star or heart shape. A configuration of this kind can be obtained by means of an extrusion head such as that of figure 2, duly designed.

According to a further aspect, there may be at least three makeup products with interface surfaces with at least one planar part, possibly irregular or in which the interface surfaces are planar, possibly irregular. An example of the said configuration is shown in figures 8A and 8C.

According to a further aspect, makeup products are cosmetic pastes such as those described in the relevant chapters above. They can be extruded or co-extruded according to one or more of the methods described. In particular, the extrusion or co-extrusion can be obtained by means of an apparatus 500, like the one described.

According to yet another aspect, the cosmetic products can be extruded or co-extruded cosmetic pastes (as described above, for example with the apparatus 500), shaped like a spheroid (for example using the device 450), dried and subsequently processed (for example using the machine 100) to eliminate a crust formed in the drying step.

According to another aspect, the cosmetic products can be extruded or co-extruded cosmetic pastes (as above), shaped like intermediate elements C, different from spheroids (for example as shown in figure 3A), dried and subsequently processed (for example using the machine 100) to eliminate a crust formed during the drying step and to give them a spheroidal shape.

The present invention also relates to a makeup product processing device 450 (figures 5 and 6) comprising:
- a first plate provided with first mutually adjacent channels with parallel axes 433, of semicircle section, and
- a second plate provided with second mutually adjacent channels with parallel axes 433, also of semicircle section, the first and the second channels being configured such that, when the first plate and the second plate are overlapped with their respective channels facing each other, each pair of first and second channels forms a tubular element with circular section;
- guides (420) configured to allow the movement of a plate relative to the other, in a direction parallel with respect to the axes of the channels are envisaged between the first and the second plate; and
- at least one movement system 430 of a plate relative to the other.

Preferentially, all the channels have an identical shape.

In one aspect, the movement system comprises a handle associated with one of the two plates, for manual movement.

According to another aspect, the movement system is automatic and comprises a kinematic mechanism adapted to move a plate relative to the other, when they are overlapped, with a reciprocating motion.

According to one aspect of the present invention, the kinematic mechanism comprises a connecting rod-crank system driven by a motor, or a pneumatic piston.

According to a further aspect of the present invention, an automatic opening system is envisaged, configured to move away the two plates in a loading step of a makeup product to be processed.

According to a further aspect, the device 450 envisages an unloading system of the makeup products processed between the two plates.

The device 450 can be used for obtaining a spheroidal makeup element by means of a method comprising the steps of:
- extruding (for example with an apparatus 500, or with an extruder or co-extruder as shown in figures 1, 2 or 7) at least one makeup paste and placing it on a plate in an angled direction, preferably orthogonal, with respect to the axes of the channels;
- overlapping the first and the second plate with the respective channels mutually facing, such as to section the extruded paste present between the plates;
- mutually moving the first and the second plate in a direction parallel to the axis of the channels, so as to shape the extruded paste present in each tubular element in the shape of a bead;
- moving away the first and the second plate to unload the beads;
- drying the beads, for example in the manner explained in the relevant chapter;
- optionally performing a second shaping (for example using the machine 100) of the beads, at least to remove a surface crust formed during the drying step.

According to an aspect of the method, the relative movement of one plate relative to the other is obtained with reciprocating motion.

The present invention also relates to a makeup product processing machine 100, for example the one shown in figure 3B. It comprises a cup-shaped body 102, 120 and a motor associated to the cup-shaped body to rotate it around an axis of symmetry thereof, the cup-shaped body defining a compartment 118 inside which a plurality of makeup products C can be housed to be processed, the internal surface of the cup-shaped body, which delimits the said compartment, being at least partially formed by an abrasive surface.

According to an aspect, the abrasive surface is perforated to remove the dust that forms during processing.

According to another aspect, the abrasive surface is a wire mesh.

Advantageously, the rotation axis is inclined with respect to a horizontal plane, so as to prevent the outflow of the material being processed from an opening of the cup-shaped body.

In an example, the cup-shaped body is a basket 120 with a cylindrical or frustum-shaped side wall.

According to one aspect, the side wall of the basket is entirely made of mesh and / or the bottom of the basket 120 is made of wire mesh.

According to another aspect, the cup-shaped body is torsionally bound to the loading mouth of a coating pan.

According to another example, the cup-shaped body 102 is a drum of a coating pan, which has a completely or at least partially abrasive surface.

The machine 100, makes available a method to produce a makeup product C and C1, comprising the following steps:
- insertion of a plurality of extruded and dried cosmetic elements C1 and C into the compartment of the cup-shaped body;
- rotation R1 of the cup-shaped body around its axis until the abrasive surface of the cup-shaped body has removed a crust from the cosmetic elements, formed during the drying step;
- removal of the cup-shaped body from the compartment.

It is also makes available a method in which the plurality of cosmetic elements C inserted in the compartment of the cup-shaped body is of a different shape from a spheroid and the rotation of the cup-shaped body is prolonged until the elements in the compartment take on a spheroidal shape.

The present invention also relates to an apparatus 500 (figures 11-14, for the production of a multi colour makeup product, comprising:
- a tank provided at a first end with an inlet and at a second end with an extrusion opening, and with a removable closure of the opening at the second end;
- a partition configured to divide the inside of the tank into at least one first and one second compartment, mutually insulated.
- a piston that slides at least partially inside the tank towards the opening at the second end;
- possibly an extrusion head with a shaped orifice, that can be combined with the opening of the second end;
- a movement system to move the piston in the tank; to extrude a paste present in the tank at least through the opening of the second end or through the extrusion head.

The apparatus can also be used in a step of the production of extruded stick-shaped pieces, having an elongated configuration and any type of section. The stick-shaped pieces obtained with the apparatus described are formed by at least two pastes of different colour and therefore they are two-coloured.

According to an aspect, the partition extends up to the opening of the second end, and preferably up to the closure, when it closes the opening.

Furthermore, the inside of the tank is defined by a first cylindrical portion, followed, in the direction of extrusion, by a second frustum-shaped convergent portion.

According to another aspect, the partition is fixed firmly in the tank, and the piston is shaped so as to be able to slide in the tank even in the presence of the partition, or, the partition is removable and is removed before operating the piston.

According to a different aspect, the apparatus comprises a tank orientation system configured to position it in at least one first position in which the mouth is facing up, and a second position wherein an axis of the tank is inclined with respect to the vertical plane, preferably in horizontal position.

The present invention also makes available a method to produce a multi colour makeup product, which can be obtained with the apparatus 500, comprising the following steps:
- preparation of a tank provided at one first end with an inlet and at one second end with an opening at the level of a convergent part of the tank, and temporary closing of the opening at the second end;
- the tank being divided into at least one first and one second compartment 510, mutually insulated;
- positioning of the tank with the first end upwards;
- after having positioned the tank, pouring a first makeup paste into the first compartment, and a second makeup paste into the second compartment 501, the first and the second makeup pastes being visually different;
- insertion of a piston in the input mouth, the said piston sliding at least partially inside the tank and opening of the second end, possibly combining it with an extrusion head;
- moving the piston relative to the tank so as to extrude, at least through the opening of the second end or the extrusion head, the cosmetic pastes present inside the cup-shaped body.
- carrying out of a first shaping of the extrusion (as described above);
- drying the extrusion (as described above);
- if necessary, performing a second shaping of the dried extrusion (as described in the relevant chapter).

According to one aspect, the tank is divided into at least two compartments by a removable partition, wherein the following further steps are envisaged:
- before pouring the first and second cosmetic pastes, inserting the partition into the tank to form one first and one second compartment, and
- before inserting the sliding piston in the mouth of the tank, removing the partition from the tank.

According to another aspect, the inside of the tank is defined by a first cylindrical portion, followed, in the direction of extrusion, by a second frustum-shaped convergent portion.

According to a further aspect, the extrusion head with an output section of a given shape may be permanently combined with the opening of the second end of the tank.

According to an aspect, prior to extruding the pastes, the tank is positioned with a substantially horizontal axis thereof.

Various embodiments of innovation have been disclosed herein, but further embodiments may also be conceived using the same innovative concept.

## Claims

1. Processing device of a makeup product comprising:
- a first plate provided with first mutually adjacent channels with parallel axes, of semicircle section, and
- a second plate provided with second mutually adjacent channels with parallel axes, also of semicircle section, the first and the second channels being configured such that, when the first plate and the second plate are overlapped with their respective channels facing each other, each pair of first and second channels forms a tubular element with circular section;
- the device having guides configured to allow the movement of a plate relative to the other, in a direction parallel with respect to the axes of the channels; and
- and at least one movement system of a plate relative to the other.

2. Device according to the preceding claim, wherein the movement system comprises a handle associated with one of the two plates, for manual movement.

3. Device according to the preceding claim, wherein the movement system is automatic and comprises a kinematic mechanism adapted to move a plate relative to the other, when they are overlapped.

4. Device according to the preceding claim, wherein the kinematic mechanism comprises a connecting rod-crank system driven by a motor, or a pneumatic piston.

5. Device according to one or more of the preceding claims, comprising an automatic opening system configured to move away the two plates in a loading step of a makeup product to be processed.

6. Device according to one or more of the preceding claims, comprising an unloading system of the makeup products processed between the two plates.

7. Method for obtaining a spheroidal makeup element by means of the one device according to one or more of the preceding claims, comprising the steps of:
- extruding at least one makeup paste and placing it on a plate in an angled direction, preferably orthogonal, with respect to the axes of the channels;
- overlapping the first and the second plate with the respective channels mutually facing, such as to section the extruded paste present between the plates;
- mutually moving the first and the second plate in a direction parallel to the axis of the channels, so as to shape the extruded paste portion present in each tubular element in the shape of a bead;
- moving away the first and the second plate to unload the beads;
- drying the beads;
- optionally performing a second shaping of the beads, at least to remove a surface crust formed during the drying step.

8. Method according to claim 7, wherein the relative movement of one plate relative to the other is reciprocating motion.
